(19) **Europäisches Patentamt / European Patent Office / Office européen des brevets**

(11) **EP 1 386 661 B1**

(12) **EUROPEAN PATENT SPECIFICATION**

(45) Date of publication and mention
of the grant of the patent:
**02.05.2012 Bulletin 2012/18**

(21) Application number: **02714502.8**

(22) Date of filing: **05.04.2002**

(51) Int Cl.:
**B01J 23/88** (2006.01)    **C07C 253/26** (2006.01)
**C07C 255/08** (2006.01)    **B01J 37/00** (2006.01)

(86) International application number:
**PCT/JP2002/003446**

(87) International publication number:
**WO 2002/083302 (24.10.2002 Gazette 2002/43)**

(54) **METHOD FOR PRODUCING MOLYBDENUM-BISMUTH-IRON CONTAINING COMPOSITE OXIDE FLUID BED CATALYST**

VERFAHREN ZUR HERSTELLUNG VON MOLYBDÄN, BISMUT UND EISEN ENTHALTENDEM MISCHOXID-WIRBELSCHICHTKATALYSATOR

PROCEDE DE PRODUCTION D'UN CATALYSEUR POUR LIT FLUIDISÉ À BASE D'OXYDES MIXTES CONTENANT DU FER, DU BISMUT ET DU MOLYBDENE

(84) Designated Contracting States:
**DE ES GB NL**

(30) Priority: **13.04.2001 JP 2001115732**

(43) Date of publication of application:
**04.02.2004 Bulletin 2004/06**

(73) Proprietor: **Dia-Nitrix Co., Ltd.**
**Tokyo 104-0031 (JP)**

(72) Inventors:
• **TAGAWA, Yuichi,**
**c/o Dia-Nitrix Co., Ltd.**
**Yokohama-shi, Kanagawa-ken 230-0053 (JP)**
• **MIYAKI, Kenichi,**
**c/o Dia-Nitrix Co., Ltd.**
**Yokohama-shi, Kanagawa-ken 230-0053 (JP)**

• **MORI, Kunio,**
**c/o Mitsubishi Rayon Co., Ltd.,**
**Chemicals Development Laboratories**
**Kanagawa-ken 230-0053 (JP)**

(74) Representative: **HOFFMANN EITLE**
**Patent- und Rechtsanwälte**
**Arabellastraße 4**
**81925 München (DE)**

(56) References cited:
EP-A- 0 337 779    EP-A1- 0 340 909
JP-A- 63 250 357    JP-A- 2001 187 772
JP-A- 2001 187 772    US-A- 3 686 138

Remarks:
The file contains technical information submitted after the application was filed and not included in this specification

**Description**

TECHNICAL FIELD

The present invention relates to a method for producing a molybdenum-bismuth-iron containing composite oxide fluid bed catalyst. Specifically, the present invention relates to a method for producing a molybdenum-bismuth-iron containing composite oxide fluid bed catalyst, which is used for an ammoxidation of many kinds of organic compounds.

BACKGROUND ART

Many catalysts, which are suitably used in methods for producing an ammoxidation product by ammoxidation of olefins, are disclosed in numerous documents. Examined Japanese Patent Application, Second Publication No. Sho 38-17967 discloses an oxide catalyst containing molybdenum, bismuth, and iron. Examined Japanese Patent Application, Second Publication No. Sho 38-19111 discloses an oxide catalyst containing iron and antimony. After these oxide catalysts were improved diligently, many improved oxide catalysts were suggested. For example, Examined Japanese Patent Application, Second Publication No. Sho 51-33888, Unexamined Japanese Patent Application, First Publication No. Sho 55-56839, Examined Japanese Patent Application, Second Publication No. Sho 58-2232, Examined Japanese Patent Application, Second Publication No. Sho 61-26419, Unexamined Japanese Patent Application, First Publication No. Hei 07-47272, Unexamined Japanese Patent Application, First Publication No. Hei 10-43595, Unexamined Japanese Patent Application, First Publication No. Hei 04-118051, and the like disclose solutions for improving an oxide catalyst by adding molybdenum, bismuth, iron, and other components and by adding iron, antimony, and other components.
In addition, in order to improve a yield of a target product, methods for producing an oxide catalyst have been examined. For example, Unexamined Japanese Patent Application, First Publication No. Hei 06-9530 discloses examples showing a method in which a slurry is heated at 90°C for three hours. Japanese Patent 2,640,356 and Unexamined Japanese Patent Application, First Publication No. Hei 01-265068 disclose a method in which a pH of a slurry is adjusted to 5 or less and it is heated to 50-120°C. Japanese Patent 2,747,920, Unexamined Japanese Patent Application, First Publication No. Hei 02-251250, Unexamined Japanese Patent Application, First Publication No. 2000-5603, Unexamined Japanese Patent Application, First Publication No. 2000-344724, Unexamined Japanese Patent Application, First Publication No. 2000-37631, and Unexamined Japanese Patent Application, First Publication No. 2000-42414 disclose a method in which a pH of a slurry is adjusted to 6 or more and it is heated to 50-120°C.
As disclosed in Examined Japanese Patent Application, Second Publication No. Sho 58-8895, in chemical products, which are produced by an oxidation or an ammoxidation of olefins, marvelous economical effects can be obtained by increasing only 1% of a yield thereof. Due to this, research to improve the catalysts is constantly being performed.
Conventional catalysts gradually improve a yield of target ammoxidation products. However, conventional catalysts do not achieve sufficient yield. In addition, it has been an objective to produce a catalyst having a high activity at high yield and an excellent reproducibility, as one of serious problems to be solved.

DISCLOSURE OF INVENTION

As a result of conducting diligent research, the present inventors have demonstrated that a catalyst which yields an objective ammoxidation product, having a high activity at high yield and an excellent reproducibility, can be produced by concentrating a slurry containing specified metal elements under specified conditions.
In other words, the present invention relates to a method for producing a molybdenum-bismuth-iron containing composite oxide fluid bed catalyst which is a method for producing a composite oxide catalyst containing components (1) molybdenum, (2) bismuth, (3) iron, (4) nickel, (5) at least one element selected from lithium, sodium, potassium, rubidium, cesium and thallium, and (6) silica, as essential components, wherein a slurry containing at least components (1), (2), (3), and (6) is subjected to a concentration treatment in a range of 50-120°C, so that the concentration difference of the slurry between before and after the concentration treatment is in a range of 2-15% by mass.

MODES FOR CARRYING OUT THE INVENTION

The present invention is explained in detailed below.
In order to produce the fluid bed catalyst in the present invention, in a method for producing a composite oxide catalyst containing components (1) molybdenum, (2) bismuth, (3) iron, (4) nickel, (5) at least one element selected from lithium, sodium, potassium, rubidium, cesium and thallium, and (6) silica, as essential components, a slurry containing at least components (1), (2), (3), and (6) is subjected to a concentration treatment in a range of 50-120°C, so that the concentration difference of the slurry between before and after the concentration treatment is in a range of 2-15% by mass. If one of these requirements is not satisfied, the objects of the present invention cannot be achieved.

The concentration treatment denotes a step in which a slurry concentration after a concentration treatment is increased by evaporating moisture contained in the slurry. In the concentration treatment, an evaporation rate of moisture is preferably adjusted. In order to adjust the evaporation rate, a reflux apparatus may be used. In addition, the concentration treatment may be also carried out while the evaporation amount of moisture is adjusted by adding water.

The slurry, which is subjected to the concentration treatment, must contain at least components (1), (2), (3), and (6). The catalyst, which produces a high ammoxidation product yield, is produced with an excellent reproducibility by concentrating the slurry containing components (1), (2), (3), and (6). Moreover, it is not necessary for the slurry to contain the required total amount of these components in the concentration treatment; a part of amount of the component may be added to the slurry after the concentration treatment.

The reasons for improving the yield of the ammoxidation products by the concentration treatment are not clear. However, it is believed that by the concentration treatment, a compound or a precursor which is suitable for improving the catalyst activity is formed. Otherwise, it is also believed that precipitates in the slurry become finer in good progress and the slurry is stabilized. Due to this, it is believed that a catalyst having superior properties is produced with a good reproducibility.

A slurry temperature in the concentration treatment is in a range of 50-120°C, and preferably in a range of 90-120°C. When the slurry temperature is less than 50°C, there are cases in which sufficient effects cannot be obtained. When it exceeds 120°C, the effects can be obtained, but the system used for the concentration treatment must be pressurized, and this is not economical. During the concentration treatment, the pressure may be reduced, normal, or increased. However, the concentration treatment is preferably carried out under reduced pressure or normal pressure. In particular, normal pressure is more preferable economically.

The duration of the concentration treatment is not restricted, in particular. In general, the concentration treatment is carried out for 30 minutes or longer, and more preferably it is carried out for 1-10 hours. The concentration treatment, of which the duration exceeds 24 hours, is not preferably in production efficiency.

The concentration difference of the slurry between before and after the concentration treatment is in a range of 2-15% by mass, and preferably in a range of 3-14% by mass. The slurry concentration denotes the ratio of mass of stabilized oxides, which are final products when the component elements contained in the slurry are changed into stabilized oxides, with respect to the total mass of the slurry. When the concentration difference of the slurry before and after the concentration treatment is less than 2% by mass, sufficient effects cannot be obtained. In contrast, when it exceeds 15% by mass, the viscosity of the slurry increases and there is a possibility that a problem may be generated in a spray-drying step after the concentration treatment. Due to this, the slurry concentration after the concentration treatment is in a range of 15-35% by mass and preferably in a range of 16-30% by mass.

Conditions of the slurry, which is subjected to the concentration treatment, is not restricted. However, the slurry, of which the pH is adjusted in a range of 1-4, is preferable. In the slurry having pH of 1-4, silica sol, which is used as a raw material of silica, is in a metastable state. Almost all of silica sol is in a liquid phase. In conventional methods for producing the catalyst, there are cases in which conditions of a spray-drying step which is necessary to produce a catalyst having sufficient particle strength to practical use, are restricted. However, according to the present invention, when the slurry having a pH of 1-4 is used, by conducting the concentration treatment, not only the yield of ammoxidation products is improved, but also the particle strength is improved. Therefore, in the present invention, the restriction to conditions of a spray-drying step decreases, and productivity of catalyst is improved.

The reasons for improving the particle strength by the concentration treatment are not clear. However, it is believed that by the concentration treatment, a crystal growth of silica or a cross-linking formation in silica is expedited.

When a pH of the slurry is relatively low, a method disclosed in Japanese Patent 2,640,356 can be adopted. In contrast, when a pH of the slurry is relatively high, a method disclosed in Japanese Patent 2,747,920 can be adopted. In addition, in order to prevent the slurry from gelling, a chelating agent, such as ethylene diamine tetra-acetic acid, lactic acid, citric acid, tartaric acid, gluconic acid, and the like, can be added in the slurry as disclosed in Japanese Patent 2,747,920. When a small amount of the chelating agent is added to the slurry having a relatively low pH, such as 1-3, the effects for preventing the gellation of slurry are sometimes obtained.

The composition of the catalyst, which is produced by the present invention, contains components (1) molybdenum, (2) bismuth, (3) iron, (4) nickel, (5) at least one element selected from lithium, sodium, potassium, rubidium, cesium and thallium, and (6) silica, as essential components. In particular, of the present invention is used to produce the catalyst represented by the following formula.

$$Mo_{10} Bi_a Fe_b Ni_c (FeSbd)_e F_f G_g H_h M_m X_x Y_y O_i (SiO_2)_j$$

In the formula, Mo, Bi, Fe, Ni, and (FeSbd) denote molybdenum, bismuth, iron, nickel, and iron antimonate. F denotes at least one element selected from yttrium, lanthanum, cerium, praseodymium, neodymium, samarium, aluminum, and gallium. G denotes at least one element selected from magnesium, calcium, strontium, barium, chromium, manganese, cobalt, copper, zinc, and cadmium. H denotes at least one element selected from titanium, zirconium, vanadium, niobium, tantalum, tungsten, germanium, tin, lead, and antimony. M denotes at least one element selected from ruthenium, rhodium, palladium, rhenium, osmium, iridium, platinum, and silver. X denotes at least one element selected from boron, phosphorus, and tellurium. Y denotes at least one element selected from lithium, sodium, potassium, rubidium, cesium, and thallium. O denotes oxygen. $SiO_2$ denotes silica. Symbols a, b, c, d, e, f, g, h, m, x, y, i, and j denote atomic ratio. When the atonic ratio of Mo is 10, a is in a range of 0.2-1.5. and preferably in a range of 0.3-1.2; b is in a range of 0.7-15, and preferably in a range of 0.8-13; c is in a range of 3-12, and preferably in a range of 4-10; d is in a range of 0.8-2, and preferably in a range of 0.9-1.5; e is in a range of 0-20; f is in a range of 0.1-1.5, and preferably in a range of 0.2-1.4; g is in a range of 0-3; h is in a range of 0-5; m is in a range of 0-1.0; x is in a range of 0-3; y is in a range of 0.05-1.5, and preferably in a range of 0.08-1.2; i is a number of oxygen element in metal oxides, which are formed by combining these elements; and j is in a range of 20-200, and preferably in a range of 30-150.

Raw materials of these elements contained in the catalyst are not restricted. For example, the raw material of the molybdenum component contains molybdenum oxides such as molybdenum trioxide; molybdic acid and molybdic acid salt such as ammonium paramolybdate, and ammonium methmolybdate; heteropoly acid containing molybdenum, such as phosphomolybdic acid, and silicomolybdic acid and heteropoly acid salt thereof.

The raw material of the bismuth component includes, for example, salts of bismuth, such as bismuth nitrate, bismuth carbonate, bismuth sulfate, bismuth acetate; bismuth trioxide; metal bismuth, and the like. These raw materials of bismuth component can be used as a solid as it is, an aqueous solution, a nitrate solution, or a slurry containing a bismuth compound, which is produced from the aqueous solution or the nitrate solution. However, bismuth nitrates, a solution containing bismuth nitrates, and a slurry produced from the solution are preferably used as the raw material of the bismuth component.

The raw material of the iron component includes, for example, ferrous oxide, ferric oxide, triiron tetroxide, ferrous nitrate, ferric nitrate, iron sulfate, iron chloride, organic acid salt of iron, iron hydroxide, and the like, in addition to a solution which is produced by solving metal iron in hot nitric acid.

The raw material of the nickel component includes, for example, nickel nitrate, nickel hydroxide, nickel oxide, and the like.

The raw material of the silica component includes, for example, silica sol, humid silica, and the like. However, silica sol is conveniently used. In general, the raw material of the other elements includes, oxides, and nitrates, carbonates, organic acid salts, hydroxides, and mixture thereof, which are changed into oxides by calcination.

When iron antimonate is contained in the catalyst, it is preferable to prepare iron antimonate and to mix with molybdenum and other components, and thereby a slurry is produced. Iron antimonate is represented by chemical formula $FeSbO_4$, as disclosed in Unexamined Japanese Patent Application, First Publication Nos. Hei 04-118051 and Hei 10-231125. The presence of iron antimonate can be confirmed by an X-ray diffraction analysis. Many methods for producing the iron antimonate have been suggested. For example, the method may be selected from the methods disclosed in Unexamined Japanese Patent Application, First Publication Nos. Hei 04-118051 and Hei 10-231125. Iron antimonate may contain a small amount of elements other than antimony and iron. The presence of the iron antimonate improves the selectivity of ammoxidation products and properties of the fluid bed catalyst.

The slurry produced by the concentration treatment in the present invention is changed to the objective fluid bed catalyst by spray drying and calcining.

A spray dryer used for spray drying the slurry produced by these processes includes, for example, ordinal rotary-disc type spray dryers, and ordinal nozzle type spray dryers, and the like. The catalyst having an objective particle diameter, which is suitable for a fluid bed catalyst, is produced by adjusting the conditions of the spray drying. After drying, the dried product is calcined at 200-500°C, and this is further calcined at 500-700°C for 0.1-20 hours. The calcining is preferably carried out in an oxygen containing gas atmosphere. The calcining is conveniently carried out in air, but this may be carried out in a mixed gas containing oxygen, nitrogen, carbon dioxide gas, water vapor, and the like. Box furnaces, tunnel calciners, rotation calciners, fluidized bed calciners, and the like may be used for the calcining. Among these, fluidized bed calciners are preferably used.

The particle diameter of the fluid bed catalyst produced by these processes is in a range of 5-200 μm, and preferably in a range of 10-150 μm.

The catalyst produced by the present invention may be used for an ammoxidation of organic compounds. The organic compound used for an ammoxidation using the catalyst produced by the present invention includes, for example, olefins, alcohols, ethers, aromatic compounds, heteroaromatic compounds, and the like. Specifically, the organic compound includes, for example, propylene, isobutene, methanol, ethanol, tert-butanol, methyl tert-butylether, toluene, xylene, picoline, quinaldine, and the like. In partictuar, if the catalyst produced by the present invention is used for an ammoxidation of propylene, isobutene, methanol, and tert-butanol, preferable results are obtained.

In general, the ammoxidation is carried out under conditions in which the molar ratio between the raw organic compound/ ammonia/ air is 1/ 0.9-3/ 8-12, and reaction temperature is in a range of 370-500°C, and reaction pressure is in a range of normal pressure to 500 kPa. Apparent contact time is in a range of 0.1-20 seconds. Air is conveniently used as an oxygen source, but air, which is diluted with water vapor, nitrogen, carbon dioxide gas, saturated hydrocarbons, and the like, is also used. In addition, oxygen enriched air is also used.

When the fluid bed catalyst having a stable catalytic structure, which is produced by the present invention, is successively used for the ammoxidation, the molybdenum component is dispersed, and a yield of ammoxidation products decreases. The reaction temperature of the ammoxidation using the fluid bed catalyst exceeds 400°C. It is impossible to prevent the dispersion of molybdenum component during the ammoxidation reaction. As a method for maintaining the properties of a catalyst for a long time, a method in which a molybdenum component is added to the catalyst during reaction has been suggested in Examined Japanese Patent Application, Second Publication No. Sho 58-57422, DE 3,311,521-C2, and WO 97/33863.

In the present invention, in order to maintain high yield of ammoxidation products for a long time, it is preferable to add a molybdenum component during an ammoxidation reaction. The molybdenum component, which is added to the catalyst during reaction, includes, for example, metal molybdenum, molybdenum trioxide, molybdic acid, ammonium dimolybdate, ammonium paramolybdate, ammonium octamolybdate, ammonium dodecamolybdate, phosphomolybdic acid, and the like. These molybdenum components can be used as gas or liquid. However, in practice, the molybdenum component in a solid state is used. In addition, these molybdenum components may be supported with inactivated materials or the catalyst. In particular, a method, in which the molybdenum component is enriched in the catalyst, is effective. Since this method has excellent utilization efficiency, and prevent generation of the problems due to a deposition of molybdenum oxide in the reaction system, this method is preferable. A method for producing molybdenum enriched catalyst disclosed in Unexamined Japanese Patent Application, First Publication No. Hei 11-33400 and the like, can be adopted.

The molybdenum component is added successively or intermittently into a reactor. The timing of addition and the amount added may be determined depending on a reaction progress. However, the amount added of the molybdenum component at a time is preferably in a range of 0.05-2% by mass with respect to 100% by mass of the catalyst, which has already added. When an excess amount of the molybdenum component is added at a time, the molybdenum component is dispersed out of the reaction system, and this is wasted. In addition, an excess amount of the molybdenum component is deposited in the reactor, and this may lead to problems in operation of the reactor.

Below, the present invention will be explained with Examples and Comparative Examples.

Catalyst Activity Test

A catalyst activity test was carried out utilizing an ammoxidation of propylene as follows. The results of the activity test are shown in Tables 1-3 and 1-4 below.

The catalyst was filled in a fluid bed reactor comprising a catalyst fluidized portion having an inner diameter of 25 mm and a height of 400 mm. After that, a mixture gas containing propylene, ammonia, air, and water vapor (mixing molar ratio of propylene/ ammonia/ air/ water vapor is 1/ 1.2/ 9.5/ 0.5) was introduced into the fluid bed reactor such that a gas linear velocity was 4.5 cm/sec. The reaction pressure was adjusted to 200 kPa.

During the reaction, a catalyst in which the molybdenum component was enriched, was added such that 0.1-0.2% by mass of molybdenum contained in the added catalyst with respect to 100% by mass of the filled catalyst was added at intervals of 100-500 hours.

$$\text{Contact time (sec.)} = \text{Catalyst volume (ml) based on apparent bulk density} / \text{Gas flow rate (ml/ sec.) converted by reaction conditions}$$

$$\text{Yield (\%) of acrylonitrile} = \text{Molar number of produced acrylonitrile} / \text{Molar number of supplied propylene} \times 100$$

Catalyst Strength Test

Strength of the catalyst particles produced in Examples and Comparative Examples was measured according to a method disclosed in Unexamined Japanese Patent Application, First Publication No. Hei 9-70542 as follows, and the strength of the catalyst particles are shown in Tables 1-3 and 1-4 below as a compressive strength (N/ particle).
Use device: Shimazu MCTM-200 (marketed by Shimazu Corporation)
Pressure element: upper pressure element, which is made of diamond and which has a flat end surface having a diameter

of 500 μm, and lower pressure plate, which is made of SUS
Pressure ratio: 7.06 X 10⁻³ N/sec
Sample: catalyst having a particle diameter of 45-50 μm

The catalyst having a particle diameter of 45-50 μm was prepared by sieving using Micro-Mesh® Precision Sieves marketed by Buckbee-Mears St. Paul Company. Thirty particles, which were randomly selected from particles having a diameter of 45-50 μm, were used as samples. The average compressive strength of the thirty particles was the compressive strength of the sample.

Example 1

A fluid bed catalyst of which the composition is represented by Mo10 Bi0.4 Fel.2 Ni6.0 Ce0.3 Cr0.8 P0.1 B0.1 K0.2 Oi (SiO₂)35 (i, which is an atomic ratio of oxygen, is determined depending on a valence of other elements, and therefore, the atomic ratio of oxygen will be omitted below) was produced by the following processes.

409.4 g of ammonium paramolybdate was dissolved in 300 g of pure water. After that, 2.7 g of 85%-phosphoric acid and 0.5 g of boric anhydride were further added. A solution, in which 45.0 g of bismuth nitrate, 4.7 g of potassium nitrate, 404.7 g of nickel nitrate, 74.2 g of chromium nitrate, 30.2 g of cerium nitrate, and 25.0 g of citric acid were added to 270 g of 3.3%-nitric acid, was mixed with the prepared solution. After that, 2438.6 g of 20%-silica sol was also added. A solution, in which 112.4 g of iron nitrate and 25.0 g of citric acid were dissolved in 270 g of pure water, was prepared, and this solution was also added. While the prepared slurry was stirred, 15%-aqueous ammonia was added to adjust the pH of the slurry to a pH of 2.0. The concentration of the slurry at this time was 21.1% by mass. This slurry was concentrated at 102°C for 7 hours such that the slurry concentration was 28.8% by mass.

The concentrated slurry was spray dried using a rotary-disc type spray dryer in that the inlet temperature was 330°C and the outlet temperature was 160°C. After dried particles were heat treated at 250°C for 2 hours and 400°C for 2 hours, and then these particles were finally calcined at 650°C for 3 hours.

Example 2

A fluid bed catalyst was prepared in a manner identical to that of Example 1. except that the composition of the prepared fluid bed catalyst was represented by Mo10 Bi0.5 Fe1.1 Ni4.0 Ce0.4 La0.2 Cr1.0 Co2.0 P0.2 B0.2 K0.2 Si35 and the prepared particles were calcined under conditions which are shown in Table 1-3. Moreover, nitrates of La and Co were used as a raw material of La and Co and they were added next to nickel nitrate.

Example 3

A fluid bed catalyst was prepared in a manner identical to that of Example 1, except that the composition of the prepared fluid bed catalyst was represented by Mo10 Bi0.5 Fe1.3 Ni5.0 Ce0.3 Cr0.6 Mg1.0 Zr0.2 K0.2 Si35 and the prepared particles were calcined under conditions which are shown in Table 1-3. Moreover, nitrates of Mg and Zr were used as a raw material of Mg and Zr and they were added next to nickel nitrate.

Example 4

A fluid bed catalyst was prepared in a manner identical to that of Example 1, except that the composition of the prepared fluid bed catalyst was represented by Mo10 Bi0.3 Fe1.1 Ni6.0 Ce0.2 Pr0.1 Cr0.8 Zn0.2 P0.3 K0.1 Rb0.1 Si40 and the prepared particles were calcined under conditions which are shown in Table 1-3. Moreover, nitrates of Pr, Zn and Rb were used as a raw material of Pr, Zn, and Rb and they were added next to nickel nitrate.

Example 5

A fluid bed catalyst was prepared in a manner identical to that of Example 1, except that the composition of the prepared fluid bed catalyst was represented by Mo10 Bi0.4 Fe0.9 N15.5 Ce0.4 Cr1.2 Mn0.4 W0.4 Pd0.01 P0.2 B0.2 K0.2 Si35 and the prepared particles were calcined under conditions which are shown in Table 1-3. Moreover, nitrates of Mn and Pd were used as a raw material of Mn and Pd and they were added next to nickel nitrate. Ammonium paratungstate was used as a raw material of W and this was added next to ammonium paramolybdate.

Example 6

A fluid bed catalyst the composition of which is represented by Mo10 Bi0.4 Fe1.2 Ni6.0 (FeSb1.1)3 Ce0.3 Cr0.8 P0.1 B0.1 K0.2 Si35, was produced by the following processes.

348.9 g of ammonium paramolybdate was dissolved in 300 g of pure water. After that, 2.3 g of 85%-phosphoric acid and 0.4 g of boric anhydride were further added. A solution, in which 38.4 g of bismuth nitrate, 4.0 g of potassium nitrate, 344.9 g of nickel nitrate, 63.3 g of chromium nitrate, 25.7 g of cerium nitrate, and 25.0 g of citric acid were added to 270 g of 3.3%-nitric acid, was mixed with the prepared solution. After that, 2078.6 g of 20%-silica sol was also added. A solution, in which 95.8 g of iron nitrate and 25.0 g of citric acid were dissolved in 270 g of pure water, was prepared, and this solution was also added. While the prepared slurry was stirred, 15%-aqueous ammonia was added to adjust the pH of the slurry to a pH of 2.3. The concentration of the slurry at this time was 21.1% by mass. This slurry was concentrated at 102°C for 7 hours such that the slurry concentration was 28.8% by mass. After that, 369.1 g of 40%-iron antimonate slurry was added.

The concentrated slurry was spray dried using a rotary-disc type spray dryer, and the inlet temperature was 330°C and the outlet temperature was 160°C. After dried particles were heat treated at 250°C for 2 hours and 400°C for 2 hours, these particles were finally calcined at 650°C for 3 hours.

Example 7

A fluid bed catalyst of which the composition is identical to the composition of the fluid bed catalyst of Example 3 was produced by the following processes.

409.8 g of ammonium paramolybdate was dissolved in 2,000 g of pure water. A solution, in which 56.3 g of bismuth nitrate, 4.7 g of potassium nitrate, 337.6 g of nickel nitrate, 55.7 g of chromium nitrate, 30.2 g of cerium nitrate, 59.5 g of magnesium nitrate, 12.4 g of zirconium nitrate, and 25.0 g of citric acid were added to 270 g of 3.3%-nitric acid, was mixed to the prepared solution. After that, 2441.0 g of 20%-silica sol was also added. A solution, in which 121.9 g of iron nitrate and 25.0 g of citric acid were dissolved in 270 g of pure water, was prepared, and this solution was also added. While the prepared slurry was stirred, 15%-aqueous ammonia was added to adjust the pH of the slurry to a pH of 2. The concentration of the slurry at this time was 14.7% by mass. This slurry was concentrated at 102°C for 2 hours such that the slurry concentration was 21.1% by mass.

The concentrated slurry was spray dried using a rotary-disc type spray dryer, and the inlet temperature was 330°C and the outlet temperature was 160°C. After dried particles were heat treated at 250°C for 2 hours and 400°C for 2 hours, these particles were finally calcined at 650°C for 3 hours.

Example 8

A fluid bed catalyst of which the composition is identical to the composition of the fluid bed catalyst of Example 1 was produced by the following processes.

409.4 g of ammonium paramolybdate was dissolved in 3,000 g of pure water. After that, 2.7 g of 85%-phosphoric acid and 0.5 g of boric anhydride were further added. A solution, in which 45.0 g of bismuth nitrate, 4.7 g of potassium nitrate, 404.7 g of nickel nitrate, 74.2 g of chromium nitrate, 30.2 g of cerium nitrate, and 25.0 g of citric acid were added to 270 g of 3.3%-nitric acid, was mixed to the prepared solution. After that, 2438.6 g of 20%-silica sol was also added. While the prepared slurry was stirred, 15%-aqueous ammonia was added to adjust the pH of the slurry to a pH of 5.0. This slurry was heated at 99°C for 1.5 hours. A solution, in which 112.4 g of iron nitrate and 25.0 g of citric acid were dissolved in 270 g of pure water, was prepared, and this solution was also added. The concentration of the slurry at this time was 13.0% by mass and the pH thereof was 4.8. This slurry was concentrated at 102°C for 4 hours such that the slurry concentration was 22.4% by mass.

The concentrated slurry was spray dried using a rotary-disc type spray dryer, and the inlet temperature was 330°C and the outlet temperature was 160°C. After dried particles were heat treated at 250°C for 2 hours and 400°C for 2 hours, these particles were finally calcined at 640°C for 3 hours.

Example 9

A fluid bed catalyst of which the composition is represented by Mo10 Bi0.6 Fe0.7 Ni7.0 (FeSb1.1)4.5 Ce0.5 P0.2 B0.2 Te0.25 K0.6 Si 40 was produced by the following processes.

180.6 g of ammonium paramolybdate was dissolved in 1,200 g of pure water. After that, 3.9 g of 85%-phosphoric acid and 2049.1 g of 20%-silica sol were further added, in that order. A solution, in which 347.1 g of nickel nitrate, 37.0 g of cerium nitrate, 10.3 g of potassium nitrate, 25 g of citric acid, and 49.6 g of bismuth nitrate were added to 270 g of 3.3%-nitric acid, was mixed with the prepared solution. While the prepared slurry was stirred, 15%-aqueous ammonia was added to adjust the pH of the slurry to a pH of 7.7. Then, the slurry was subjected to a heat treatment in which the slurry was refluxed at 99°C for 1.5 hours.

5.4 g of metal tellurium, 4.5 g of ammonium paramolybdate, and 20 g of hydrogen peroxide were added to 250 g of water, and these were dissolved in water by stirring at 95-100°C. This prepared solution was cooled to room temperature and 25 g of citric acid and 48.2 g of iron nitrate were dissolved in the solution. While the prepared solution was stirred, aqueous ammonia was added to adjust the pH of the slurry to a pH of 9.2. 115.9 g of ammonium paramolybdate was added little by little and dissolved in the solution. After that, the pH of the solution was adjusted to 7 by adding aqueous ammonia.

This solution was mixed in the prepared slurry, and 480.8 g of 40%-iron antimonate slurry was further added. The concentration of the slurry at this time was 13.8% by mass and the pH thereof was 7.2. This slurry was concentrated at 102°C for 5.5 hours such that the slurry concentration was 17.2% by mass.

The concentrated slurry was spray dried using a rotary-disc type spray dryer, and the inlet temperature was 330°C and the outlet temperature was 160°C. After dried particles were heat treated at 250°C for 2 hours and 400°C for 2 hours, these particles were finally calcined at 580°C for 3 hours.

Example 10

A fluid bed catalyst of which the composition is identical to the composition of the fluid bed catalyst of Example 1 was produced by the following processes.

409.4 g of ammonium paramolybdate was dissolved in 3,000 g of pure water. After that, 2.7 g of 85%-phosphoric acid and 0.5 g of boric anhydride were further added. A solution, in which 45.0 g of bismuth nitrate, 4.7 g of potassium nitrate, 404.7 g of nickel nitrate, 74.2 g of chromium nitrate, 30.2 g of cerium nitrate, and 25.0 g of citric acid were added to 270 g of 3.3%-nitric acid, was mixed with the prepared solution. After that, 2438.6 g of 20%-silica sol were further added. Another solution, in which 112.4 g of iron nitrate and 25.0 g of citric acid were dissolved in 270 g of pure water, was prepared, and this solution was also added. While the prepared slurry was stirred, 15%-aqueous ammonia was added to adjust the pH of the slurry to a pH of 3.0. This slurry was heated at 99°C for 1.5 hours. The concentration of the slurry at this time was 12.5% by mass. This slurry was concentrated at 102°C for 4 hours such that the slurry concentration was 22.1% by mass.

The concentrated slurry was spray dried using a rotary-disc type spray dryer, and the inlet temperature was 330°C and the outlet temperature was 160°C. After dried particles were heat treated at 250°C for 2 hours and 400°C for 2 hours, these particles were finally calcined at 650°C for 3 hours.

Comparative Example 1

A comparative fluid bed catalyst was prepared in a manner identical to that of Example 1, except that the concentration treatment for 7 hours was not carried out and the prepared particles were calcined under conditions which are shown in Table 1-4.

Comparative Example 2

A comparative fluid bed catalyst having a composition identical to that of Example 3 was prepared in a manner identical to that of Example 7, except heat treatment at 99°C for 2 hours was carried out instead of concentration treatment and the prepared particles were calcined under conditions which are shown in Table 1-4.

Comparative Example 3

A comparative fluid bed catalyst having a composition identical to that of Example 6 was prepared in a manner identical to that of Example 6, except that the concentration treatment for 7 hours was not carried out and the prepared particles were calcined under conditions which are shown in Table 1-4.

Comparative Example 4

A comparative fluid bed catalyst having a composition identical to that of Example 1 was prepared in a manner identical to that of Example 8, except that the solution containing iron nitrate and citric acid was added to the slurry after the concentration treatment and the prepared particles were calcined under conditions which are shown in Table 1-4.

The ammoxidation of propylene was carried out under conditions explained above using the catalysts prepared in Examples and Comparative Examples.

The results are shown in Tables below.

Table 1-1

| | | Mo | Bi | Fe | Ni | FeSb (Fe / Sb) | F | G | H | M | X | Y | SiO$_2$ |
|---|---|---|---|---|---|---|---|---|---|---|---|---|---|
| Example | 1 | 10 | 0.4 | 1.2 | 6.0 | | Ce 0.3 | Cr 0.8 | | | P 0.1  B 0.1 | K 0.2 | 35 |
| | 2 | 10 | 0.5 | 1.1 | 4.0 | | Ce 0.4  La 0.2 | Cr 1.0  Co 2.0 | | | P 0.2  B 0.2 | K 0.2 | 35 |
| | 3 | 10 | 0.5 | 1.3 | 5.0 | | Ce 0.3 | Cr 0.6  Mg 1.0 | Zr 0.2 | | | K 0.2 | 35 |
| | 4 | 10 | 0.3 | 1.1 | 6.0 | | Ce 0.2  Pr 0.1 | Cr 0.8  Zn 0.2 | | | P 0.3 | K 0.1  Rb 0.1 | 40 |
| | 5 | 10 | 0.4 | 0.9 | 5.5 | | Ce 0.4 | Cr 1.2  Mn 0.4 | W 0.4 | Pd 0.01 | P 0.2  B 0.2 | K 0.2 | 35 |
| | 6 | 10 | 0.4 | 1.2 | 6.0 | 3.0  3.3 | Ce 0.3 | Cr 0.8 | | | P 0.1  B 0.1 | K 0.2 | 35 |
| | 7 | 10 | 0.5 | 1.3 | 5.0 | | Ce 0.3 | Cr 0.6  Mg 1.0 | Zr 0.2 | | | K 0.2 | 35 |
| | 8 | 10 | 0.4 | 1.2 | 6.0 | | Ce 0.3 | Cr 0.8 | | | P 0.1  B 0.1 | K 0.2 | 35 |
| | 9 | 10 | 0.6 | 0.7 | 7.0 | 4.5  5 | Ce 0.5 | | | | P 0.2  B 0.2  Te 0.25 | K 0.6 | 40 |
| | 10 | 10 | 0.4 | 1.2 | 6.0 | | Ce 0.3 | Cr 0.8 | | | P 0.1  B 0.1 | K 0.2 | 35 |

Table 1-2

| | | Mo | Bi | Fe | Ni | FeSb | | F | G | | H | M | X | | Y | SiO$_2$ |
|---|---|---|---|---|---|---|---|---|---|---|---|---|---|---|---|---|
| | | | | | | Fe | Sb | | | | | | | | | |
| Comparative Example | 1 | 10 | 0.4 | 1.2 | 6.0 | | | Ce 0.3 | Cr 0.8 | | | | P 0.1 | B 0.1 | K 0.2 | 35 |
| | 2 | 10 | 0.5 | 1.3 | 5.0 | | | Ce 0.3 | Cr 0.6 | Mg 1.0 | Zr 0.2 | | | | K 0.2 | 35 |
| | 3 | 10 | 0.4 | 1.2 | 6.0 | 3.0 | 3.3 | Ce 0.3 | Cr 0.8 | | | | P 0.1 | B 0.1 | K 0.2 | 35 |
| | 4 | 10 | 0.4 | 1.2 | 6.0 | | | Ce 0.3 | Cr 0.8 | | | | P 0.1 | B 0.1 | K 0.2 | 35 |

Table 1-3

| Examples | | Concentration Treatment | | | | Burning temperature [°C] | Contact time [sec.] | Yield of acrylonitrile [%] | | | Compressive strength X $10^{-3}$ [N/particle] |
|---|---|---|---|---|---|---|---|---|---|---|---|
| | | pH before concentration treatment | Concentration before concentration treatment [%] | Concentration after concentration treatment [%] | Concentration treatment time [hours] | | | Progress time [hour] | | | |
| | | | | | | | | 50 | 500 | 1,000 | |
| | 1 | 2.2 | 21.1 | 28.8 | 7.0 | 650 | 2.4 | 82.9 | 82.9 | 83.0 | 150 |
| | 2 | 2.5 | 21.1 | 28.7 | 7.0 | 650 | 2.3 | 82.6 | 82.6 | 82.5 | 150 |
| | 3 | 2.3 | 21.0 | 28.6 | 7.0 | 640 | 2.4 | 82.8 | 82.6 | 82.7 | 135 |
| | 4 | 1.8 | 21.1 | 28.7 | 7.0 | 660 | 2.1 | 82.5 | 82.6 | 82.5 | 140 |
| | 5 | 2.0 | 21.0 | 28.7 | 7.0 | 650 | 2.3 | 82.6 | 82.4 | 82.3 | 145 |
| | 6 | 2.3 | 21.1 | 28.7 | 7.0 | 650 | 2.2 | 83.1 | 83.0 | 83.1 | 160 |
| | 7 | 2.0 | 14.7 | 21.1 | 2.0 | 640 | 2.7 | 82.5 | 82.3 | 82.3 | 120 |
| | 8 | 2.3 | 12.6 | 22.0 | 4.0 | 640 | 2.3 | 82.8 | 82.6 | 82.6 | 155 |
| | 9 | 7.2 | 13.8 | 17.2 | 5.5 | 580 | 2.3 | 83.1 | 82.9 | 82.7 | 195 |
| | 10 | 3.0 | 12.5 | 22.1 | 4.0 | 650 | 2.4 | 82.7 | 82.6 | 82.6 | 145 |

Table 1-4

| | | Concentration Treatment | | | | Burning temperature [°C] | Contact time [sec.] | Yield of acrylonitrile [%] | | | Compressive strength X 10⁻³ [N/particle] |
|---|---|---|---|---|---|---|---|---|---|---|---|
| | | | | | | | | Progress time [hour] | | | |
| | | pH before concentration treatment | Concentration before concentration treatment [%] | Concentration after concentration treatment [%] | Concentration treatment time [hours] | | | 50 | 500 | 1,000 | |
| Comparative Examples | 1 | - | no concentration | | 0.0 | 630 | 3.3 | 81.6 | 81.4 | 81.1 | 80 |
| | | | 21.1 | 21.1 | | | | | | | |
| | 2 | - | no concentration | | heating 2.0 | 640 | 3.1 | 81.5 | 81.5 | 81.3 | 100 |
| | | | 14.7 | 14.7 | | | | | | | |
| | 3 | - | no concentration | | 0.0 | 630 | 2.5 | 82.0 | 81.7 | 81.6 | 85 |
| | | | 21.1 | 21.1 | | | | | | | |
| | 4 | 4.8 | 13.0 | 22.4 | 4.0 | 640 | 2.4 | 81.8 | 81.7 | 81.8 | 90 |

INDUSTRIAL APPLICABILITY

The production method of the present invention can yield a molybdenum-bismuth-iron containing composite oxide fluid bed catalyst with an excellent reproducibility. The molybdenum-bismuth-iron containing composite oxide fluid bed catalyst produced by the present invention has a high activity and this yields the target ammoxidation product at high yield. For example, by using the molybdenum-bismuth-iron containing composite oxide fluid bed catalyst produced by the present invention in an ammoxidation of propylene, acrylonitrile can be obtained at high yield.

**Claims**

1.   A method for producing a molybdenum-bismuth-iron containing composite oxide fluid bed catalyst which is a method for producing a composite oxide catalyst containing components (1) molybdenum, (2) bismuth, (3) iron, (4) nickel, (5) at least one element selected from lithium, sodium, potassium, rubidium, cesium and thallium, and (6) silica, as essential components,
    wherein the composite oxide fluid bed catalyst has a composition represented by Mo10 Bia Feb Nic (Fe Sbd)e Ff Gg Hh Mm Xx Yy Oi(SiO$_2$)j
    in the formula, Mo, Bi, Fe, Ni, and (FeSbd) denote molybdenum, bismuth, iron, nickel and iron antimonite; F denotes at least one element selected from yttrium, lanthanum, cerium, praseodymium, neodymium, samarium, aluminum, and gallium; G denotes at least one element selected from magnesium, calcium, strontium, barium, chromium, manganese, cobalt, copper, zinc, and cadmium; H denotes at least one element selected from titanium, zirconium, vanadium, niobium, tantalum, tungsten, germanium, tin, lead, and antimony; M denotes at least one element selected from ruthenium, rhodium, palladium, rhenium, osmium, iridium, platinum, and silver; X denotes at least one element selected from boron, phosphorus, and tellurium; Y denotes at least one element selected from lithium, sodium, potassium, rubidium, cesium, and thallium; O denotes oxygen; SiO$_2$ denotes silica; symbols a, b, c, d, e, f, g, h, m, x, y, i, and j denote atomic ratio; when the atomic ratio of Mo is 10, a is in a range of 0.2-1.5, b is in a range of 0.7-15, c is in a range of 3-12, d is in a range of 0.8-2, e is in a range of 0-20, f is in a range of 0.1-1.5, g is in a range of 0-5, h is in a range of 0-3, m is in a range of 0-1.0, x is in a range of 0-3, y is in a range of 0.05-1.5, i is the number of oxygen elements in metal oxides, which are formed by combining these elements; and j is in a range of 20-200, wherein a slurry containing at least components (1), (2), (3), and (6) is concentrated in a range of 50-120°C, so that the concentration difference of the slurry between before and after the concentration treatment is in a range of 2-15% by mass;
    the concentration of the slurry after the concentration treatment is in a range of 15-35% by mass; and
    the method comprises a spray-drying step after the concentration treatment.

**Patentansprüche**

1.   Verfahren zur Herstellung eines Molybdän-Bismut-Eisen enthaltenden Mischoxid-Wirbelbettkatalysators, das ein Verfahren zur Herstellung eines Mischoxidkatalysators ist, der die Komponenten (1) Molybdän, (2) Bismut, (3) Eisen, (4) Nickel, (5) mindestens ein Element ausgewählt aus Lithium, Natrium, Kalium, Rubidium, Cäsium und Thallium und (6) Siliziumdioxid als wesentliche Komponenten enthält,
    worin der Mischoxid-Wirbelbettkatalysator eine Zusammensetzung hat, die dargestellt ist durch
    Mo10 Bia Feb Nic (Fe Sbd)e Ff Gg Hh Mm Xx Yy Oi(SiO$_2$)j
    wobei in der Formel Mo, Bi, Fe, Ni und (FeSbd) Molybdän, Bismut, Eisen, Nickel und Eisenantimonit darstellen; F mindestens ein Element ausgewählt aus Yttrium, Lanthan, Cer, Praseodym, Neodym, Samarium, Aluminium und Gallium darstellt; G mindestens ein Element ausgewählt aus Magnesium, Kalzium, Strontium, Barium, Chrom, Mangan, Kobalt, Kupfer, Zink und Kadmium darstellt; H mindestens ein Element ausgewählt aus Titan, Zirkonium, Vanadium, Niob, Tantal, Wolfram, Germanium, Zinn, Blei und Antimon darstellt; M mindestens ein Element ausgewählt aus Ruthenium, Rhodium, Palladium, Rhenium, Osmium, Iridium, Platin und Silber darstellt; X mindestens ein Element ausgewählt aus Bor, Phosphor und Tellur darstellt; Y mindestens ein Element ausgewählt aus Lithium, Natrium, Kalium, Rubidium, Cäsium und Thallium darstellt; O Sauerstoff darstellt; SiO$_2$ Siliziumdioxid darstellt; die Symbole a, b, c, d, e, f, g, h, m, x, y, i und j das Atomverhältnis darstellen; wenn das Atomverhältnis von Mo 10 ist, a im Bereich von 0,2 bis 1,5 ist, b im Bereich von 0,7 bis 15 ist, c im Bereich von 3 bis 12 ist, d im Bereich von 0,8 bis 2 ist, e im Bereich von 0 bis 20 ist, f im Bereich von 0,1 bis 1,5 ist, g im Bereich von 0 bis 5 ist, h im Bereich von 0 bis 3 ist, m im Bereich von 0 bis 1,0 ist, x im Bereich von 0 bis 3 ist, y im Bereich von 0,05 bis 1,5 ist, i die Anzahl der Sauerstoffelemente in den Metalloxiden ist, die durch Kombinieren dieser Elemente gebildet werden; und j im Bereich von 20 bis 200 ist,

worin eine Aufschlämmung, die zumindest die Komponenten (1), (2), (3) und (6) enthält, im Bereich von 50 bis 120°C aufkonzentriert wird, so dass die Konzentrationsdifferenz der Aufschlämmung zwischen vor und nach der Behandlung zur Aufkonzentration im Bereich von 2 bis 15 Massen% ist;
die Konzentration der Aufschlämmung nach der Behandlung zur Aufkonzentration in einem Bereich von 15 bis 35 Massen% ist; und
das Verfahren einen Sprühtrocknungsschritt nach der Behandlung zur Aufkonzentration umfasst.

## Revendications

1. Procédé pour produire un catalyseur pour lit fluidisé à base d'oxyde composite contenant du molybdène, du bismuth, du fer qui est un procédé pour produire un catalyseur à base d'oxyde composite contenant des composants de (1) molybdène, (2) bismuth, (3) fer, (4) nickel, (5) au moins un élément sélectionné parmi le lithium, le sodium, le potassium, le rubidium, le césium et le thallium et (6) silice, en tant que composants essentiels,
dans lequel le catalyseur pour lit fluidisé à base d'oxyde composite a une composition représentée par
Mo10 Bia Feb Nic (Fe Sbd)e Ff Gg Hh Mm Xx Yy Oi(SiO2)j
dans la formule, Mo, Bi, Fe, Ni et (FeSbd) désignent le molybdène, le bismuth, le fer, le nickel et l'antimoine ferreux ; F désigne au moins un élément sélectionné parmi l'yttrium, le lanthane, le cérium, le praséodyme, le néodyme, le samarium, l'aluminium et le gallium ; G désigne au moins un élément sélectionné parmi le magnésium, le calcium, le strontium, le baryum, le chrome, le manganèse, le cobalt, le cuivre, le zinc et le cadmium ; H désigne au moins un élément sélectionné parmi le titane, le zirconium, le vanadium, le niobium, le tantale, le tungstène, le germanium, l'étain, le plomb, et l'antimoine ; M désigne au moins un élément sélectionné parmi le ruthénium, le rhodium, le palladium, le rhénium, l'osmium, l'iridium, le platine, et l'argent ; X désigne au moins un élément sélectionné parmi le bore, le phosphore et le tellure ; Y désigne au moins un élément sélectionné parmi le lithium, le sodium, le potassium, le rubidium, le césium et le thallium ; O désigne l'oxygène ; $SiO_2$ désigne la silice ; les symboles a, b, c, d, e, f, g, h, m, x, y, i et j désignent un rapport atomique ; lorsque le rapport atomique de Mo est 10, a est dans une plage de 0,2 - 1,5, b est dans une plage de 0,7 - 15, c est dans une plage de 3 - 12, d est dans une plage de 0,8 - 2, e est dans une plage de 0 - 20, f est dans une plage de 0,1 - 1,5, g est dans une plage de 0 - 5, h est dans une plage de 0 - 3, m est dans une plage de 0 - 1,0, x est dans une plage de 0 - 3, y est dans une plage de 0,05 - 1,5, i est le nombre d'éléments d'oxygène dans des oxydes métalliques, qui sont formés en combinant ces éléments ; et j est dans une plage de 20 - 200,
dans lequel une pâte contenant au moins les composants (1), (2), (3) et (6) est concentrée dans une plage de 50 - 120 °C, de sorte que la différence de concentration de la pâte entre avant et après le traitement de concentration est dans une plage de 2 - 15 % en masse ;
la concentration de la pâte après le traitement de concentration est dans une plage de 15 - 35 % en masse ; et le procédé comprend une étape de séchage par atomisation après le traitement de concentration.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO3817967 B
- JP SHO3819111 B
- JP SHO5133888 B
- JP SHO5556839 B
- JP SHO582232 B
- JP SHO6126419 B
- JP HEI0747272 B
- JP HEI1043595 B
- JP HEI04118051 B
- JP HEI069530 B
- JP 2640356 B
- JP HEI01265068 B
- JP 2747920 B
- JP HEI02251250 B
- JP 2000005603 A
- JP 2000344724 A
- JP 2000037631 A
- JP 2000042414 A
- JP SHO588895 B
- JP HEI10231125 B
- JP SHO5857422 B
- DE 3311521 C2
- WO 9733863 A
- JP HEI1133400 B
- JP HEI970542 B